# EUROPÄISCHE PATENTANMELDUNG

(11) **EP 0 535 710 A1**
(43) Veröffentlichungstag der Anmeldung: **07.04.1993**
(21) Anmeldenummer: 92116965.2
(22) Anmeldetag: 05.10.1992
(51) Int. Cl.: C07D 475/04

(54) **Verfahren zur Herstellung von Erdalkalimetallsalzen von (6R)-N(10)-Formyl-5,6,7,8-tetrahydrofolsäure**

(30) Priorität: 04.10.1991 CH 2933/91
(71) Anmelder: SAPEC S.A. FINE CHEMICALS, CH-6903 Lugano (CH)
(72) Erfinder: Melera, Attilio, CH-6926 Montagnola (CH); Marazza, Fabrizio, CH-6937 Breno/TI (CH)
(74) Vertreter: Zink-Wild, Markus Peter

(57) **Zusammenfassung**

Das erfindungsgemässe Verfahren zur Herstellung von Erdalkalimetallsalzen von (6R)-N(10)-Formyl-5,6,7,8-tetrahydrofolsäure der Formel I ist dadurch gekennzeichnet, dass man zu einem in Wasser oder in einer wässrigen Pufferlösung gelösten Ammonium- oder Alkalimetallsalz von (6RS)-N(10)-Formyl-5,6,7,8-tetrahydrofolsäure der Formel II, ein Erdalkalimetallsalz entweder in der Form eines Festkörpers oder in der Form einer wässrigen Lösung hinzugibt, vorzugsweise unter einer Inertgasatmosphäre, das so erhaltene Gemisch auskristallisieren lässt, und den erhaltenen Festkörper der Formel I abtrennt.

## Beschreibung

Die vorliegende Erfindung betrifft ein Verfahren zur Herstellung von Erdalkalimetallsalzen von (6R)-N(10)-Formyl-5,6,7,8-tetrahydrofolsäure.

Verfahren zur Herstellung von reduzierten Folaten mit einheitlicher (6S)- oder (6R)-Konfiguration sind in der französischen Patentanmeldung Nr. 90 03032 mit der Publikations-Nr. 2 659 330 beschrieben. Hierin ist auch der weitere Stand der Technik beschrieben. Diese Angaben werden mit diesem Hinweis als auch hierin offenbart betrachtet.

Im folgenden Teil wird für 5,6,7,8-Tetrahydrofolsäure die Abkürzung THF verwendet.

Es ist bekannt, dass die Hydrolyse von N(5),N(10)-Methenyl-THF.Cl⁻ in einem wässrigen Puffer bei einem neutralen oder basischen pH-Wert zu N(10)-Formyl-THF führt; siehe D.R. Robinson, Methods in Enzymology (1971) Vol XVIII, Part B [184], und G.K. Smith, P.A. Benkovic, S.J. Benkovic, Biochem. (1981), 20, 4034 und J.C. Rabinowitz, Methods in Enzymology (1963), 6, 814.

Es ist auch bekannt, dass N(10)-Formyl-THF thermisch in N(5)-Formyl-THF umgewandelt werden kann; siehe den genannten Artikel von D.R. Robinson.

Hydrolisiert man ein reines Diastereoisomer von N(5),N(10)-Methenyl-THF.Cl⁻, so erhält man das entsprechende, reine Diastereoisomer von N(10)-Formyl-THF; siehe den genannten Artikel von G.K. Smith et al.

Völlig überraschend wurde nun gefunden, dass die Zugabe eines Erdalkalimetallsalzes zu einem in Wasser oder in einer wässrigen Pufferlösung gelösten Ammonium- oder Alkalimetallsalz eines Diastereoisomerengemisches von (6RS)-N(10)-Formyl-THF zur selektiven Kristallisation des Erdalkalimetallsalzes von (6R)-N(10)-Formyl-THF führt.

Das erfindungsgemässe Verfahren zur Herstellung von Erdalkalimetallsalzen von (6R)-N(10)-Formyl-5,6,7,8-tetrahydrofolsäure der Formel I 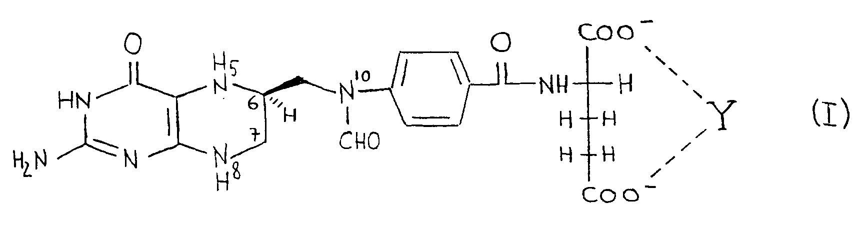
worin Y ein Erdalkalimetallkation bedeutet, und insbesondere Ca²⁺ ist, ist dadurch gekennzeichnet, dass man zu einem in Wasser oder in einer wässrigen Pufferlösung gelösten Ammonium- oder Alkalimetallsalz von (6RS)-N(10)-Formyl-5,6,7,8-tetrahydrofolsäure der Formel II 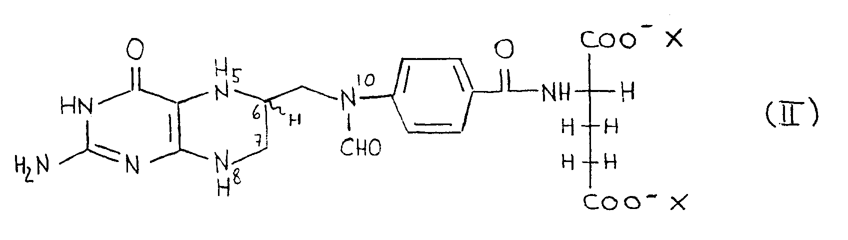
worin X ein Alkalimetallkation oder NH₄⁺ bedeutet,
ein Erdalkalimetallsalz entweder in der Form eines Festkörpers oder in der Form einer wässrigen Lösung hinzugibt, vorzugsweise unter einer Inertgasatmosphäre,
das so erhaltene Gemisch auskristallisieren lässt, und
den erhaltenen Festkörper der Formel I abtrennt.

Bevorzugte Ausführungsformen dieses Verfahrens sind in den abhängigen Ansprüchen definiert.

Zur Feststellung der diastereoisomeren Reinheit wurde die gemäss dem erfindungsgemässen Verfahren hergestellte Verbindung (6R)-N(10)-Formyl-THF.Ca²⁺ gemäss dem in der französischen Patentanmeldung Nr. 90 03032 mit der Publikations-Nr. 2 659 330 beschriebenen Verfahren in (6S)-N(5)-Methyl-THF umgewandelt. Die Analyse an einer chiralen HPLC-Säule (RESOLVOSIL-Säule) ergab eine diastereoisomere Reinheit von 80 bis 90 %.

Die erfindungsgemäss hergestellten diastereoisomerenreinen Verbindungen können gemäss dem oben genannten Artikel von D.R. Robinson in (6S)-N(5)-Formyl-THF umgewandelt werden.

Die erfindungsgemäss hergestellten diastereoisomerenreinen Verbindungen können auch gemäss bekannten Verfahren unter sauren Bedingungen in (6R)-N(5),N(10)-Methenyl-THF umgewandelt werden; siehe den oben genannten Artikel von J.C. Rabinowitz.

(6R)-N(5),N(10)-Methenyl-THF kann gemäss bekannten Verfahren zu (6S)-N(5)-Methyl-THF reduziert werden; siehe White, Bailey, Goldman, J. Biol. Chem. (1978), 253, 242.

Das nachfolgende Beispiel illustriert die vorliegende Erfindung.

### Beispiel

Zu 200 ml einer wässrigen 0,2 N Lösung von Tris-hydroxymethyl-amino-methan-hydrochlorid, welche mit Salzsäure auf einen pH-Wert von 8,3 eingestellt worden ist, wurden unter Rühren bei Raumtemperatur und unter einer Argonatmosphäre gleichzeitig und portionenweise 15 g (6RS)-N(5),N(10)-Methenyl-THF.Cl⁻ und genügend 20%-ige wässrige NaOH zugegeben, um eine klare Lösung mit einem pH-Wert von 8,3 zu erhalten. Diese Lösung wurde auf eine Temperatur von 30°C erwärmt, 3,7 g CaCl₂.2H₂O wurden in wenig Wasser (etwa 10 ml) gelöst und zur oben genannten Lösung hinzugegben.

Nach einigen Minuten setzte eine spontane Kristallisation ein. Das Gemisch wurde bei der gleichen Temperatur während 2 Stunden gerührt. Der kristalline Festkörper wurde abfiltriert, einmal mit Wasser, einmal mit 94%-igem Ethanol und einmal mit Aceton gewaschen und unter reduziertem Druck bei einer Temperatur von 60°C getrocknet.

Man erhielt 5,5 g an weissen Kristallen von (6R)-N(10)-Formyl-THF.Ca²⁺.

Die HPLC-Analyse an einer reversed phase Säule zeigte eine 98%-ige chemische Reinheit.

MS [FAB/Matrix: Thioglyzerin]: m⁺: 512, entsprechend der Summenformel C₂₀H₂₁N₇O₇Ca.

IR (KBr): 3407, 3345, 3220, 1660, 1635, 1605, 1575, 1545, 1505, 1455, 1425, 1410, 1360, 1330 cm⁻¹.

Zur Feststellung der diastereoisomeren Reinheit wurden 15 mg der oben genannten Kristalle in 1,5 ml einer 1,8 %-igen Lösung von p-Toluolsulfonsäure gelöst. Danach wurden 6 mg NaBH₃CN zugegeben und das Gemisch wurde während 30 Minuten bei Raumtemperatur gerührt. Dabei wurde (6R)-N(10)-Formyl-THF vollständig in (6S)-N(5)-Methyl-THF umgewandelt. Die Analyse der letzteren Verbindung mittels einer chiralen HPLC-Säule (RESOLVOSIL) zeigte eine diastereoisomere Reinheit von 85 %.

## Patentansprüche

1. Verfahren zur Herstellung von Erdalkalimetallsalzen von (6R)-N(10)-Formyl-5,6,7,8-tetrahydrofolsäure der Formel I 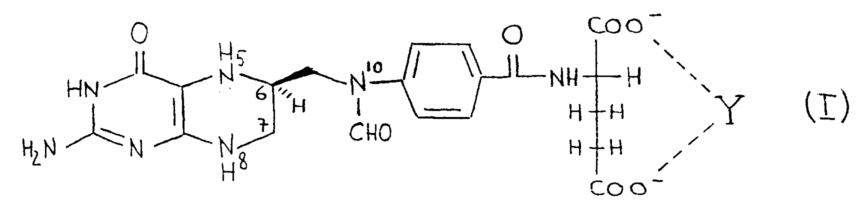 worin Y ein Erdalkalimetallkation bedeutet, und insbesondere Ca²⁺ ist,
dadurch gekennzeichnet, dass man zu einem in Wasser oder in einer wässrigen Pufferlösung gelösten Ammonium- oder Alkalimetallsalz von (6RS)-N(10)-Formyl-5,6,7,8-tetrahydrofolsäure der Formel II 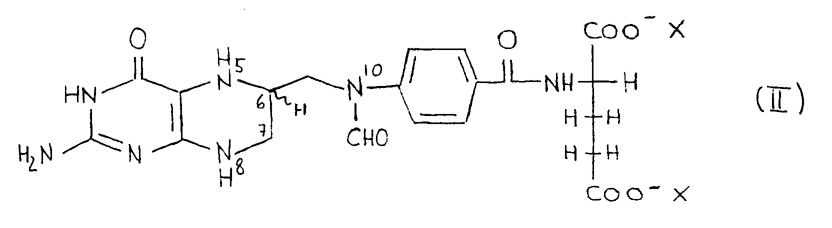 worin X ein Alkalimetallkation oder NH₄⁺ bedeutet,
ein Erdalkalimetallsalz entweder in der Form eines Festkörpers oder in der Form einer wässrigen Lösung hinzugibt, vorzugsweise unter einer Inertgasatmosphäre,
das so erhaltene Gemisch auskristallisieren lässt, und
den erhaltenen Festkörper der Formel I abtrennt.

2. Verfahren nach Anspruch 1, dadurch gekennzeichnet, dass das verwendete Wasser deionisiertes Wasser und/oder entgastes Wasser ist.

3. Verfahren nach einem der Ansprüche 1 bis 2, dadurch gekennzeichnet, dass der verwendete Puffer keine Fällung mit einem Erdalkalimetallsalz ergibt, und vorzugsweise Tris-hydroxymethyl-amino-methan-hydrochlorid ist.

4. Verfahren nach einem der Ansprüche 1 bis 3, dadurch gekennzeichnet, dass man das Erdalkalimetallsalz bei einer Temperatur von 20°C bis 60°C, vorzugsweise bei 30°C, hinzugibt.

5. Verfahren nach einem der Ansprüche 1 bis 4, dadurch gekennzeichnet, dass man die Kristallisation bei einer Temperatur von 20°C bis 40°C, vorzugsweise bei 30°C, durchführt.

6. Verfahren nach einem der Ansprüche 1 bis 5, dadurch gekennzeichnet, dass das verwendete Erdalkalimetallsalz ein Erdalkalimetallhalogenid, vorzugsweise ein Calciumhalogenid, insbesondere Calciumchlorid, oder ein Erdalkalimetallacetat ist.

7. Verwendung der Verbindungen der Formel I als Ausgangsmaterialien zur Herstellung von (6R)-N(5),N(10)-Methenyl-THF, (6S)-N(5)-Methyl-THF und (6S)-N(5)-Formyl-THF.
